Europäisches Patentamt

European Patent Office

Office européen des brevets

(19)

(11) Numéro de publication: **0 211 783**
A1

(12)

# DEMANDE DE BREVET EUROPEEN

(21) Numéro de dépôt: 86430025.6

(22) Date de dépôt: 04.07.86

(51) Int. Cl.⁴: **A 61 B 1/04**
G 02 B 23/26, G 01 C 11/06

(30) Priorité: 12.07.85 FR 8510848

(43) Date de publication de la demande:
25.02.87 Bulletin 87/9

(84) Etats contractants désignés:
AT BE CH DE FR GB IT LI LU NL SE

(71) Demandeur: Société en Commandite par Actions dite:
Société Générale de Remorquage et de Travaux
Maritimes CHAMBON et Cie.
148, rue Sainte
F-13007 Marseille(FR)

(72) Inventeur: Buisson, Jean-Pierre
10, rue des Tilleuls Lotissement Jas Neuf
F-13260 Carry-le-Rouet(FR)

(72) Inventeur: Mutius, Bernard
168, rue Abbé de l'Epée
F-13005 Marseille(FR)

(74) Mandataire: Azais, Henri et al,
c/o CABINET BEAU DE LOMENIE 14, rue Raphael
F-13008 Marseille(FR)

(54) Endoscope stéréoscopiques.

(57) Un endoscope stéréoscopique comporte deux conduits optiques identiques (1d, 1g). Chaque conduit est équipé d'une caméra de télévision (5d, 5g) qui est connectée sur un magnétoscope (6d, 6g). L'appareil comporte deux écrans vidéo (7d, 7g), sur lesquels on peut faire apparaître en synchronisme les couples d'images filmées simultanément par les deux caméras. Un dispositif binoculaire (8d, 8g) permet de regarder simultanément les deux images pour percevoir une vue en relief. Un micro-ordinateur (10) permet de traiter les images vidéo, de les numériser et de mesurer les dimensions d'un défaut selon trois directions trirectangulaires.

Une application est l'observation en relief de tumeurs ou autres tissus parasitaires dans le corps humain.

EP 0 211 783 A1

./...

Fig. 1

La présente invention a pour objet des endoscopes stéréoscopiques.

On utilise dans le domaine industriel,médical ou vétérinaire des endoscopes qui comportent un conduit optique, rigide ou souple, qui est composé par exemple d'une suite de lentilles coaxiales ou d'un faisceau de fibres optiques et qui est suffisamment fin pour être introduit dans des cavités de l'organisme,par exemple dans l'oesophage ou dans le colon.

La plupart des endoscopes connus à ce jour comportent un oculaire qui permet d'observer une cible avec un seul oeil et des moyens pour éclairer celle-ci en lumière visible.

On connaît également des endoscopes stéréoscopiques comportant deux conduits optiques parallèles et un oculaire stéréoscopique permettant de percevoir une image en relief.

La demande de brevet japonais JP - A. 59 204 018(SUMITOMO DENKI KOGYO K.K.) résumée dans les patents abstracts of Japan Vol. 9 No. 72 (P-345) décrit des endoscopes stéréoscopiques comportant deux conduits optiques pour la formation des images, un guide de lumière, des réflecteurs situés en regard d'une fenêtre latérale située à l'extrémité distale et un oculaire stéréoscopique.

La demande de brevet FR. A. 2.013.417 (R. WOLF) décrit des endoscopes stéréoscopiques comportant deux conduits optiques parallèles, des fibres conductrices de lumière et une chambre noire permettant d'obtenir des paires de photographies.

Un objectif de l'invention est de procurer des endoscopes stéréoscopiques qui permettent de transformer deux images simultanées d'une même cible vue sous deux angles différents en signaux vidéo qui peuvent être soit transmis à distance, soit enregistrés puis transportés à distance sous forme de cassettes pour restituer les deux images sur deux écrans afin de percevoir une image en relief en regardant les deux écrans par un dispositif binoculaire.

Un autre objectif de l'invention est de permettre d'associer un endoscope à des moyens électroniques de traitement des signaux qui permettent d'améliorer la qualité des images et qui permettent d'obtenir des valeurs numériques qui donnent les trois dimensions d'une cible suivant trois axes trirectangulaires.

Les endoscopes stéréoscopiques selon l'invention comportent de façon connue, deux conduits optiques identiques et parallèles qui

2

délivrent deux images d'une même cible.

Les objectifs de l'invention sont atteints au moyen d'endoscopes stéréoscopiques qui comportent, en outre, deux caméras de télévision qui filment en synchronisme respectivement chacune des deux images, deux écrans vidéo sur chacun desquels apparaissent en synchronisme respectivement les deux images filmées simultanément par les deux caméras et un dispositif binoculaire stéréoscopique qui permet à un observateur de regarder les deux écrans afin de percevoir une image en relief de la cible.

Avantageusement, un endoscope selon l'invention comporte, en outre, deux magnétoscopes qui enregistrent en synchronisme respectivement les signaux vidéo fournis par chacune des deux caméras et des moyens pour faire apparaître en synchronisme sur lesdits écrans vidéo les images enregistrées simultanément par les deux magnétoscopes.

Selon un mode de réalisation préférentiel, un endoscope selon l'invention comporte, en outre, une unité centrale de calcul qui est reliée par une unité d'interface à la sortie des deux magnétoscopes et qui est programmée pour numériser des couples d'images simultanées sélectionnées par un opérateur, pour mesurer les coordonnées sur chacune des deux images d'un couple de points sélectionnés par ledit opérateur par rapport à un repère orthonormé propre à chacune des deux images, pour ramener ces coordonnées à un même repère orthonormé, pour calculer les équations dans ce repère orthonormé commun des quatre droites reliant chacun des deux points respectivement à ses deux images dans chaque plan image et pour calculer les coordonnées desdits points définis chacun comme l'intersection de deux droites.

Un endoscope stéréoscopique selon l'invention comporte, de préférence, une unité périphérique du type imprimante alphagraphique ou traceur qui est associée à ladite unité centrale électronique pour reproduire des copies sur papier des images à partir des valeurs numériques enregistrées dans la mémoire de l'unité centrale et pour imprimer sur ces copies les dimensions mesurées suivant trois axes trirectangulaires de chaque cible.

Selon une variante, un endoscope stéréoscopique selon l'invention comporte deux lames semi-transparentes qui sont disposées obliquement par rapport aux axes des deux conduits optiques et il comporte, en outre, un émetteur de rayonnement qui envoie sur lesdites lames semi-réfléchissantes un rayonnement ultraviolet ou un faisceau laser, lequel rayonnement est réfléchi partiellement dans lesdits conduits optiques.

L'invention a pour résultat de nouveaux endoscopes stéréoscopiques.

Selon une variante, un endoscope stéréoscopique selon l'invention comporte à son extrémité distale deux capteurs d'image à l'état solide délivrant des signaux électriques numériques. Ces signaux sont disponibles à l'autre extrémité de l'endoscope pour traitement ultérieur ou restitution d'images vidéo. Ledit endoscope comporte, en outre, un ensemble de fibres optiques permettant l'éclairement de la scène inspectée à partir d'une source lumineuse et ceci pour un spectre semblable à celui déjà défini.

L'invention a pour résultat de nouveaux endoscopes stéréoscopiques.

Les endoscopes selonl'invention équipés d'un appareil binoculaire pour regarder simultanément avec les deux yeux deux images d'une même cible vue sous deux angles différents, permettent de percevoir une vue en relief de la cible et d'apprécier par exemple la hauteur d'une tumeur ou la profondeur d'un ulcère ou bien, dans des applications industrielles, d'apprécier la profondeur d'une crique ou d'une fissure ou la profondeur de pénétration de la corrosion dans un métal.

Les endoscopes selon l'invention équipés de deux caméras de télévision permettent de transmettre des signaux vidéo à distance par exemple dans un poste d'observation central où l'on peut faire apparaître en synchronisme les images prises par les deux caméras sur deux écrans vidéo et observer ces deux écrans avec les deux yeux au moyen d'un appareil binoculaire, afin de percevoir une vue en relief.

Par exemple, un plongeur portant deux caméras de télévision dans le dos et tenant à la main un endoscope stéréoscopique selon l'invention relié aux deux caméras, peut accéder avec l'extrémité de l'endoscope à des points difficilement accessibles, par exemple à des soudures situées dans les noeuds d'une structure métallique ou à des points situés à l'arrière d'une pièce mécanique où l'on ne peut accéder.Il peut également approcher l'extrémité de l'endoscope très près d'un point qui nécessite une observation minutieuse pour obtenir un effet de loupe et pour éviter la turbidité de l'eau.

Les caméras peuvent être également portées par un engin sous-marin, habité ou non.

Dans ces applications sous-marines, les caméras sont reliées par câble à une salle d'observation située sur un support de surface (navire ou plateforme), dans lequel se trouve un opérateur devant deux écrans vidéo qu'il observe au moyen d'un binoculaire. L'observateur perçoit en temps réel une image en relief et il transmet des instructions au plongeur.

Les endoscopes selon l'invention équipés de deux caméras de télévision et de deux magnétoscopes permettent d'enregistrer les signaux vidéo, puis d'observer les images en temps différé et de ne conserver que celles qui sont utiles. On peut transporter les cassettes vidéo pour effectuer le traitement des images dans un poste de contrôle éloigné. Par exemple, on peut enregistrer des images en mer, puis transporter les cassettes à terre pour observer et traiter les images dans une station terrestre équipée à cet effet.

En associant les deux magnétoscopes à la fois à deux écrans vidéo et à une unité centrale de traitement électronique, par exemple à un micro-ordinateur, un opérateur, qui regarde défiler les images sur les écrans vidéo, peut sélectionner des couples d'images, déclencher une transformation analogique à numérique du couple d'images sélectionné avec enregistrement des valeurs numériques puis il peut déplacer un curseur horizontalement et verticalement entre deux points extrêmes d'un défaut pour obtenir une mesure numérique des dimensions maxima de ce défaut suivant deux axes $x$ et $y$ situés dans le plan perpendiculaire aux axes des conduits optiques de l'endoscope.

Il peut également, grâce à un programme enregistré, obtenir une mesure de la hauteur ou de la profondeur d'un défaut suivant un troisième axe $z$ perpendiculaire au plan des axes $x$ et $y$.

Cette mesure de la dimension $z$ est très importante pour apprécier la gravité des défauts aussi bien dans les applications médicales qu'industrielles et l'obtention de cette mesure est possible grâce à la vision stéréoscopique qui permet à un opérateur de déterminer les deux points extrêmes d'un défaut dans le sens de l'axe $z$.

Un endoscope selon l'invention équipé d'un émetteur de rayonnements ultraviolets ou laser permet d'associer l'observation en relief d'un défaut à une irradiation de celui-ci sans avoir à déplacer l'endoscope.

Un endoscope selon l'invention équipé de deux caméras à infrarouges permet d'observer des images thermographiques en relief d'une cible interne à un organisme vivant.

La description suivante se réfère aux dessins annexés qui représentent, sans aucun caractère limitatif, des exemples de réalisation de l'invention.

La figure 1 est une représentation schématique d'un premier mode de réalisation d'un endoscope stéréoscopique selon l'invention.

La figure 2 est une représentation schématique d'une variante de réalisation d'un endoscope stéréoscopique selon l'invention.

La figure 3 est une vue géométrique.

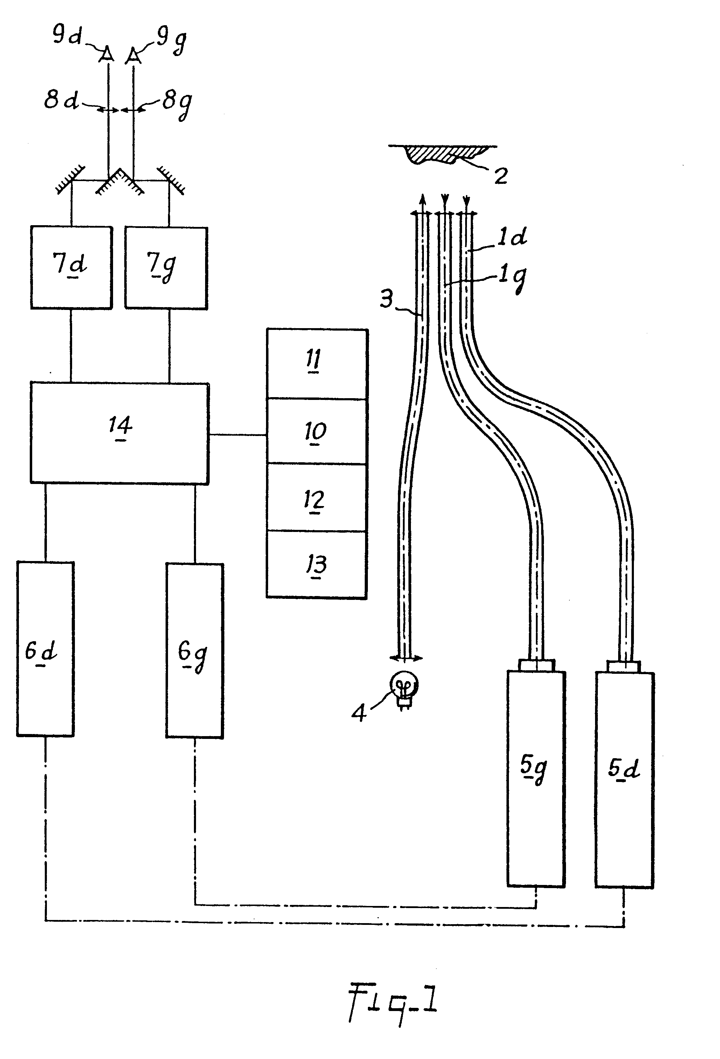

La figure 1 représente un endoscope stéréoscopique qui comporte deux conduits optiques identiques 1d, 1g rigides ou souples, chaque conduit optique étant composé, de façon connue, soit d'une série de lentilles coaxiales, soit de fibres optiques, soit de barreaux rigides.

La figure 1 représente un endoscope en cours d'utilisation pour observer une cible 2 qui peut être par exemple un tissu parasitaire, par exemple une tumeur, un kyste, un polype etc.... dans le cas d'une application médicale ou vétérinaire ou qui peut être par exemple une crique ou une fissure dans le cas d'une application industrielle.

Dans de nombreux cas, il est intéressant de pouvoir évaluer non seulement le contour de la cible, mais également son épaisseur ou sa profondeur. Par exemple, dans le cas de défauts métalliques tels que des fissures ou des cavités dues à la corrosion, la profondeur est un élément d'appréciation essentiel de la gravité d'un défaut. Dans les applications médicales, la hauteur ou l'épaisseur d'une tumeur permet d'apprécier l'importance de celle-ci.

L'objectif de l'invention est de procurer des endoscopes qui permettent de percevoir une image en relief d'une cible afin d'apprécier les dimensions de celle-ci dans trois directions trirectangulaires.

La figure 1 représente un endoscope qui comporte deux caméras 5d, 5g qui sont placées,respectivement à la sortie des deux conduits optiques 5g, 5d.Les deux caméras 5d, 5g peuvent être des caméras vidéo normales qui convertissent la lumière visible en un signal vidéo.

Selon une variante, les caméras 5d, 5g sont des caméras à infrarouges et, dans ce cas, les conduits optiques 1d, 1g sont composés de matériaux transparents aux infrarouges, par exemple de lentilles et de barreaux en fluorure de lithium, de calcium ou de magnésium qui sont transparents aux radiations comprises entre 0,1 µ et 14 µ.

Selon cette variante, les caméras infrarouges convertissent les radiations infrarouges émises par la cible en signaux vidéo. Les deux caméras 5d et 5g sont synchronisées.

La figure 1 représente un exemple de réalisation d'un endoscope qui comporte un troisième conduit optique 3 qui transmet à la cible les radiations émises par un émetteur 4.

L'émetteur 4 peut être une source de lumière visible destinée à éclairer la cible et, dans ce cas, le conduit optique 3 peut être un conduit en fibres optiques qui est transparent aux radiations visibles.

L'émetteur 4 peut également comporter un émetteur de rayonnement ultraviolet et, dans ce cas, le conduit optique 3 est composé de matériaux transparents aux ultraviolets.

L'émetteur 4 peut également comporter un émetteur laser. Dans ce cas, on peut d'abord repérer une cible en lumière visible, mesurer ses dimensions puis, sans déplacer l'endoscope, la traiter par un faisceau laser.

Chaque caméra est associée à un magnétoscope, respectivement 6d, 6g qui enregistre les signaux vidéo délivrés par la caméra.

Le dispositif comporte, en outre, deux écrans vidéo 7d, 7g, sur chacun desquels ont peut afficher, soit en temps réel, soit en temps différé, les images enregistrées respectivement dans le magnétoscope 6d ou 6g. Lors de l'affichage, les deux magnétoscopes sont synchronisés, de sorte que les couples d'images apparaissant simultanément sur les deux écrans 7d, 7g correspondent respectivement à deux images filmées par les deux caméras 5d, 5g entre les mêmes impulsions de synchronisation de trame.

L'appareil comporte, en outre, un dispositif binoculaire comportant deux oculaires 8d, 8g distants d'un écartement égal à celui des deux yeux 9d, 9g d'un opérateur qui peut ainsi regarder les deux écrans en superposant les couples d'images simultanées pour percevoir une image en relief pendant la prise de vues et également pendant le traitement informatique des images.

Grâce à la présence de deux caméras vidéo, de deux magnétoscopes et de deux écrans vidéo, le mode de réalisation selon la figure 1 permet d'enregistrer les signaux vidéo et, de ce fait, il permet de reproduire les images en temps différé pour mieux les étudier. On peut alors supprimer toutes les images inutiles et conserver uniquement celles sur lesquelles la cible apparaît le mieux pour leur faire subir un traitement électronique qui sera décrit ci-après.

La présence de deux caméras vidéo permet également de transmettre les signaux vidéo à distance.

Par exemple, un endoscope selon l'invention peut être utilisé pour inspecter des ouvrages sous-marins afin de vérifier le bon état de ceux-ci. Dans ce cas, l'endoscope est couplé à deux caméras sous-marines et les signaux vidéo sortant des caméras sont transmis par un ombilical à un support de surface qui porte les équipements électroniques nécessaires à l'enregistrement des images, à leur visualisatiosn en relief et au traitement des signaux.

La présence de deux magnétoscopes permet d'enregistrer les signaux vidéo sur cassettes vidéo et de transporter ces cassettes pour les reproduire sur d'autres magnétoscopes faisant partie d'une unité centrale de traitement électronique.

Les signaux vidéo enregistrés dans les deux magnétoscopes subissent plusieurs traitements qui sont expliqués ci-après.

Le traitement commence par la sélection d'un ou plusieurs couples d'images prises simultanément par les deux caméras et sur lesquelles la cible apparaît le plus nettement. Ce choix est fait par un opérateur qui examine les images sur les écrans 7d et 7g des moniteurs vidéo et qui peut arrêter le défilement des deux magnétoscopes sur un couple d'images. Les magnétoscopes ont de bonnes qualités de stabilité de la vitesse de défilement, de manière à pouvoir assurer une bonne synchronisation entre eux.

Ils disposent également d'un arrêt sur image très précis. Avantageusement, les magnétoscopes comportent un générateur d'horloge qui permet de les synchroniser et d'inscrire en clair sur la bande vidéo la date et l'heure des enregistrements.

Les couples d'images sélectionnés sont transformés en valeurs numériques par une unité centrale électronique, par exemple par un micro-ordinateur, qui est programmé pour échantillonner les signaux vidéo, par exemple à raison de 512 pixels par ligne et par colonne et pour calculer pour chaque pixel une valeur numérique exprimant le niveau de gris. Compte tenu de la sensibilité de l'oeil humain aux niveaux de gris, on peut se borner à coder chaque pixel sur six bits, ce qui correspond à 64 niveaux de gris. Les valeurs numériques des pixels sont enregistrées dans la mémoire du micro-ordinateur.

Après avoir numérisé un couple d'images, on peut effectuer sur les valeurs numériques des traitements si ces images ne présentent pas des qualités suffisantes de contraste permettant de définir le contour de la cible. Ces traitements d'images sont mis en oeuvre par un opérateur de façon adaptée à chaque cas. Ils peuvent comporter par exemple les fonctions suivantes :

- Histogramme de niveaux de gris.
- Traitement par filtres gradients ou par filtres sélectifs.
- Amélioration des contrastes.
- Extraction de contours.

La mise en oeuvre de ces traitements est faite de manière interactive par un opérateur au moyen d'un ensemble écran-clavier, l'opérateur jugeant de la validité du traitement sur l'écran du moniteur vidéo sur lequel apparaissent les couples d'images traités.

Les traitements d'image vidéo destinés à améliorer la qulité, notamment le contraste, sont des traitements connus et il n'est pas nécessaire de les décrire plus en détail.

Un endoscope selon l'invention permet de mesurer les trois dimensions X Y Z mesurées suivant trois axes trirectangulaires de n'importe quel segment reliant deux points d'une cible, lesquels points sont choisis par un opérateur. L'axe oz est parallèle à l'axe des deux conduits optiques. Les axes ox et oy sont situés dans le plan image perpendiculaire à l'axe oz.

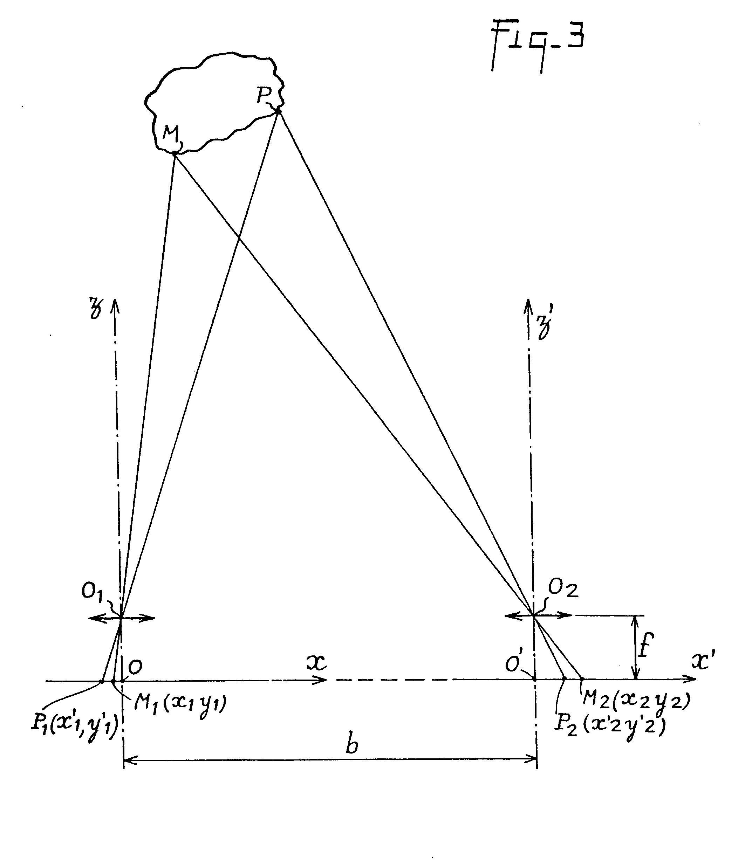

La figure 3 est une représentation géométrique permettant d'expliquer le traitement des signaux qui permet d'obtenir la mesure des trois dimensions X Y et Z d'un segment rectiligne reliant deux points M et P d'une cible. Cette figure représente les deux objectifs $O_1$, $O_2$ des deux conduits optiques, leurs axes $O_1Z$ et $O_2Z$ qui sont parallèles et distants d'une longueur b et les plans image des deux objectifs dans lesquels se forment les images respectives M1, P1 et P2, P2 des deux points M et P, lesquelles images sont converties en signaux vidéo qui peuvent être numérisés.

L'opérateur est muni d'un moyen de commande manuel permettant de déplacer un curseur dans les deux plans image en contrôlant ces déplacements à l'aide de la vision stéréoscopique, ce qui lui permet d'amener le curseur d'abord sur un premier point M bien déterminé puis sur un deuxième point P également déterminé et de déclencher un comptage de pixels mesurés suivant les axes Ox et Oy.

Il effectue ainsi un pointage double qui définit dans le plan image de la caméra vidéo de gauche deux points M1, P1 et dans le plan image de la caméra de droite deux points M2, P2.

On considère deux repères orthonormés rattachés à chaque plan image et ayant respectivement pour origine les points O et O' situés respectivement à l'intersection de l'axe $O_1Z$ et de l'axe $O_2Z$ avec le plan image correspondant.

Les coordonnées du point M1 sont x1,y1 dans le repère Oxy. Les coordonnées du point M2 sont x2,y2 dans le repère O'xy. Les coordonnées du point P1 sont x'1 y'1 dans le repère Oxy. Les coordonnées du point P2 sont x'2 y'2 dans le repère O'xy.

Etant donné que les deux repères orthonormés Oxy et O'xy se déduisent l'un de l'autre par une translation parallèle aux axes Ox et O'x de valeur b, on peut ramener les coordonnées de tous les points à un même repère orthonormé.

On choisit par exemple un repère orthonormé OXYZ d'origine O. Les coordonnées des différents points Mi et Pi (i = 1 ou 2) sont alors :

M1 : X1 = x1    Y1 = y1    Z1 = 0
P1 : M'1 = x'1    Y'1 = y'1    Z'1 = 0
M2 : X2 = x2+b    Y2 = y2    Z2 = 0
P2 : X'2 = x'2+b    Y'2 = y'2    Z2 = 0.

Les coordonnées des centres optiques 01 et 02 dans le système OXYZ sont parfaitement définies par la distance du plan image à l'objectif qui est généralement égale àla distance focale f et par l'écartement  b  entre les deux axes.

Les droites  P1 P, M1 M  passant par le centre optique  01 ainsi que les droites  P2 P et M2 M  passant par le centre optique  02 sont parfaitement définies ainsi que leur équation et on programme l'unité de calcul pour qu'elle établisse ces équations et qu'elle calcule les coordonnées dans le référentiel  OXYZ  du point  M  qui est l'intersection des droites  MM1 et MM2  et du point  P  qui est l'intersection des droites  PP1 et PP2.

A partir des trois coordonnées d'un couple de points  M et P  l'unité centrale calcule la longueur du segment  MP.

Ainsi si les points  M et P  sont choisis comme étant deux points extrêmes de la cible dans le sens de l'axe  OX  puis dans le sens de l'axe  OY, on mesure la largeur et la hauteur d'une cible.

La vision en relief permet à l'opérateur de déterminer également un point  M  qui est le plus rapproché  et un point  P  qui est le plus éloigné et de mesurer ainsi la profondeur de la cible mesurée suivant l'axe  OZ  parallèle aux axes optiques.

Une fois les calculs effectués, le micro-ordinateur garde ceux-ci en mémoire, ce qui permet de les faire apparaître sur un moniteur vidéo et/ou sur une copie sur papier de l'image d'une cible.

Cette copie sur papier est réalisée à l'aide d'une unité périphérique commandée par le micro-ordinateur, par exemple à l'aide d'un traceur ou d'une imprimante alphagraphique qui reproduit l'image à partir des valeurs numériques du signal vidéo enregistrées. Chaque copie sur papier porte des inscriptions alphanumériques permettant de repérer l'image et les trois mesures  x, y et z  du défaut apparaissant sur cette image.

La figure  1  représente schématiquement, sous forme de bloc diagramme les composants essentiels de l'unité centrale électronique de traitement des signaux vidéo à savoir une unité centrale de calcul  et de mémoire  10  qui soit un ensemble multiprocesseur programmé pour assurer à la fois des traitements des images en vue d'améliorer leur qualité, la numérisation des images et les divers traitements des valeurs numériques, soit un micro-ordinateur qui est programmé pour gérer le déroulement des différentes phases de traitement des signaux vidéo.

Le repère  11  représente un écran vidéo de contrôle. Le repère 12 représente une unité périphérique,par exemple une imprimante capable de reproduire sur papier une copie de l'image apparaissant sur l'écran 11. Le repère 13 représente un clavier de commande des opérations. Le repère 14 représente une unité d'interface entre les magnétoscopes, les deux écrans vidéo 7d, 7g et l'unité centrale 10.

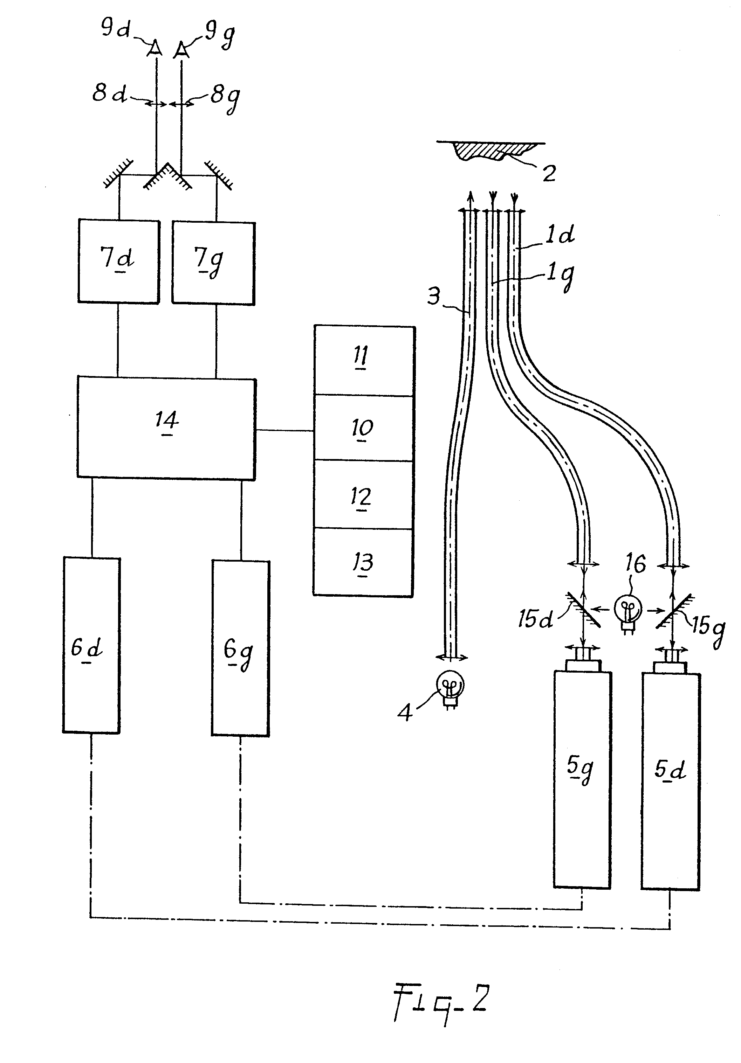

La figure 2 représente une variante de réalisation d'un endoscope stéréoscopique selon l'invention.

Les parties homologues sont représentées par les mêmes repères.

L'endoscope selon la figure 2 comporte deux lames semi-réfléchissantes 15d, 15g qui sont intercalées entre la lentille de sortie des deux conduits optiques 1d, 1g et les caméras 5d, 5g et qui sont inclinées à 45° par rapport aux axes optiques des deux conduits.

L'endoscope comporte en outre, un émetteur de rayonnement 16 qui émet un rayonnement ultraviolet (U.V.) ou laser.

Les conduits optiques 1d et 1g sont composés de matériaux transparents aux longueurs d'onde comprises entre 0,1 μ et 14 μ.

Le rayonnement U.V. ou le faisceau laser émis par l'émetteur 16 est réfléchi partiellement par les deux lames semi-réfléchissantes 15d et 15g et il est transmis jusqu'à la cible 2 par les deux conduits optiques.

L'adjonction à un endoscope stéréoscopique selon la figure 1 de deux lames semi-réfléchissantes 15d, 15g et d'un émetteur 16 de rayonnement ultraviolet permet d'éclairer une cible en lumière ultraviolette pour mieux observer certains défauts qui sont rendus fluorescents. Elle permet également d'envoyer sur une cible dont on a déjà observé l'image en relief en lumière visible un faisceau laser afin de brûler ou de cautériser la cible tout en continuant à observer l'image en relief de la cible pendant le traitement.

Un endoscope stéréoscopique selon l'invention peut être utilisé dans le domaine médical ou vétérinaire pour observer des images en relief de cibles situées à l'intérieur de l'organisme et pour produire des copies sur papier de ces images portant des indications alphanumériques permettant de repérer la position des cibles et leurs dimensions.

Un endoscope selon l'invention équipé de caméras infrarouges permet d'obtenir des thermographies de cibles intérieures à l'organisme

Selon une variante de réalisation, un endoscope stéréoscopique selon l'invention comporte à son extrémité distale deux capteurs d'images composés d'une matrice d'éléments à l'état solide qui transforment la lumière captée en signaux électriques

Ces capteurs sont reliés par des fils à l'extrémité externe de l'endoscope où les signaux sont disponibles pour un traitement ultérieur ou pour restituer une image vidéo. Cet endoscope comporte, en outre, un ensemble de fibres optiques permettant d'éclairer la cible à partir d'une source lumineuse externe.

1

REVENDICATIONS

1. Endoscope stéréoscopique du type comportant deux conduits optiques identiques (1d, 1g) qui délivrent deux images d'une même cible (2), caractérisé en ce qu'il comporte, en outre, deux caméras de télévision (5d, 5g) qui filment en synchronisme respectivement chacune des deux images, deux écrans vidéo (7d, 7g) sur chacun desquels apparaissent en synchronisme respectivement les deux images filmées simultanément par les deux caméras et un dispositif binoculaire stéréoscopique (8d, 8g) qui permet à un observateur de regarder les deux écrans afin de percevoir une image en relief de la cible (2).

2. Endoscope stéréoscopique selon la revendication 1, caractérisé en ce qu'il comporte, en outre, deux magnétoscopes (6d, 6g) qui enregistrent en synchronisme respectivement les signaux vidéo fournis par chacune des deux caméras et des moyens pour faire apparaître en synchronisme sur ledit écran ficéo les images enregistrées simultanément par les deux magnétoscopes.

3. Endoscope selon la revendication 2, caractérisé en ce qu'il comporte, en outre, une unité centrale de calcul (10) qui est reliée par une unité d'interface (14) à la sortie des deux magnétoscopes (5d, 6g) et qui est programmée pour numériser des couples d'images simultanées sélectionnées par un opérateur, pour mesurer les coordonnées sur chacune des deux images d'un couple de points (MP) sélectionnés par ledit opérateur par rapport à un repère orthonormé propre à chacune des deux images, pour ramener ces coordonnées à un même repère orthonormé, pour calculer les équations dans ce repère orthonormé commun des quatre droites reliant chacun des deux points (MP) respectivement à ses deux images (M1, M2, P1, P2) dans chaque plan image et pour calculer les coordonnées desdits points (MP) définis chacun comme l'intersection de deux droites.

4. Endoscope stéréoscopique selon la revendication 3, caractérisé en ce qu'il comporte, en outre, une unité périphérique (12) du type imprimante alphagraphique ou traceur qui est associée à ladite unité centrale électronique pour reproduire des copies sur papier des images à partir des valeurs numériques enregistrées dans la mémoire de l'unité centrale et pour imprimer sur ces copies les dimensions mesurées suivant trois axes trirectangulaires (x, y, z) de chaque cible.

5. Endoscope selon l'une quelconque des revendications 1 à 4, caractérisé en ce que lesdits conduits optiques (1d, 1g) sont composés de matériaux transparents aux longueurs d'onde comprises entre 0,1 µ et 14 µ.

6. Endoscope selon la revendicaiton 5, caractérisé en ce qu'il comporte deux caméras infrarouges (5d, 5g), de sorte qu'il permet d'observer des images thermographiques en relief d'une cible interne à l'organisme.

7. Endoscope selon la revendication 5, caractérisé en ce qu'il comporte deux lames semi-réfléchissantes (15d, 15g) qui sont disposées obliquement par rapport aux axes des deux conduits optiques (1d, 1g) et il comporte, en outre, un émetteur de rayonnement (16) qui envoie sur lesdites lames semi-réfléchissantes un rtayonnement ultraviolet ou un faisceau laser, lequel rayonnement est réfléchi partiellement dans lesdits conduits optiques.

Fig-1

2/3 0211783

Fig. 2

0211783

Fig.3

# 0211783

Numero de la demande

## Office européen des brevets

## RAPPORT DE RECHERCHE EUROPEENNE

EP 86 43 0025

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| D,X | PATENT ABSTRACTS OF JAPAN; vol. 9, no. 72 (P-345)[1795], 2 avril 1985; & JP-A-59 204 017 (SUMITOMO DENKI KOGYO K.K.) 19-11-1984 * Résumé * | 1,5,7 | A 61 B 1/04 G 02 B 23/26 G 01 C 11/06 |
| | --- | | |
| D,X | FR-A-2 013 417 (R. WOLF GmbH) * Page 1, lignes 8-25; page 2, lignes 17-35; page 3, lignes 23-38; figures 1-4 * | 1 | |
| | --- | | |
| A | FR-A-2 411 423 (MOSKOVSKY INZHENERNO-FIZICHESKY INSTITUT) * Page 4, lignes 23-40; page 8, lignes 11-20; page 12, lignes 20-29; figures 1,6 * | 1,2 | |
| | --- | | DOMAINES TECHNIQUES RECHERCHES (Int Cl 4) |
| A | EP-A-0 121 411 (K.K. TOSHIBA) * Abrégé; page 3, lignes 2-16; page 3, ligne 28 - page 4, ligne 12; page 6, ligne 33 - page 7, ligne 25; page 9, ligne 16 - page 10, ligne 8; figures 1,2 * | 2,3 | A 61 B G 02 B G 01 C |
| | --- | | |
| A | EP-A-0 095 660 (MESSERSCMITT-BÖLKOW-BLOHM GmbH) * Abrégé; page 3, lignes 13-28; page 4, lignes 13-34; page 6, lignes 19-28; page 7, lignes 14-29; page 8, lignes 19-26; figures 1-3 * | 2-4 | |
| | ---          -/- | | |

Le present rapport de recherche a été établi pour toutes les revendications

| Lieu de la recherche | Date d'achevement de la recherche | Examinateur |
|---|---|---|
| LA HAYE | 14-10-1986 | RIEB K.D. |

**RAPPORT DE RECHERCHE EUROPEENNE**

Office européen
des brevets

EP 86 43 0025

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int Cl 4) |
|---|---|---|---|
| A | DE-A-2 746 614 (E. RAPP) <br> * Page 1, revendication 1; page 2, revendications 8,10; page 6, lignes 11-25; page 7, ligne 25 - page 8, ligne 10; figures * | 5,6 | |
| A | FR-A-2 260 831 (BENDIX CORP.) <br> * Page 8, ligne 18 - page 9, ligne 21; figure 3 * | 2,7 | |
| A | DE-B-1 254 289 (M.S.A. HADI) <br> * Colonne 1, lignes 32-41; colonne 2, lignes 23-30,45-52; colonne 3, lignes 4-11; figure 1 * | 5,7 | |
| A | PATENT ABSTRACTS OF JAPAN; vol. 6, no. 125 (P-127)[1003], 10 juillet 1982; & JP-A-57 52 812 (SHIMAZU SEISAKUSHO K.K.) 29-03-1982 <br> * Résumé * | 2,4 | |

--- 

DOMAINES TECHNIQUES
RECHERCHES (Int Cl.4)

Le présent rapport de recherche a été établi pour toutes les revendications

| Lieu de la recherche <br> LA HAYE | Date d'achèvement de la recherche <br> 14-10-1986 | Examinateur <br> RIEB K.D. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82